# EUROPEAN PATENT SPECIFICATION

(11) **EP 1 314 979 B1**
(45) Date of publication and mention of the grant of the patent: **15.10.2008**
(21) Application number: 01956991.2
(22) Date of filing: 21.08.2001
(51) Int. Cl.: G01N 33/483, A61B 5/00, A61B 5/04, G01N 27/27, G01N 27/416, A61B 5/103, G01N 33/50

(54) **METHOD AND DEVICE FOR DETERMINING STATE OF SKIN SURFACE**
VERFAHREN UND VORRICHTUNG ZUM BESTIMMEN DES ZUSTANDES DER HAUTOBERFLÄCHE
PROCEDE ET DISPOSITIF PERMETTANT DE DETERMINER L'ETAT DE LA SURFACE DE LA PEAU

(30) Priority: 23.08.2000 JP 2000252732; 13.12.2000 JP 2000378581
(43) Date of publication of application: 28.05.2003
(60) Divisional application: 07008527.9
(73) Proprietor: SHISEIDO COMPANY, LTD., Chuo-ku, Tokyo 104-8010 (JP)
(72) Inventor: DENDA, Mitsuhiro, c/o Shiseido Company, Ltd., Yokohama-shi, Kanagawa 236-8643 (JP); ASHIDA, Yutaka, c/o Shiseido Company, Ltd., Yokohama-shi, Kanagawa 236-8643 (JP); INOUE, Kaori, c/o Shiseido Company, Ltd., Yokohama-shi, Kanagawa 236-8643 (JP)
(74) Representative: Merkle, Gebhard
(86) International application number: PCT/JP2001/007142
(87) International publication number: WO 2002/016933

(56) References cited:
- WO-A-00/01301
- DE-A1- 4 410 691
- JP-A- 3 162 663
- JP-A- 11 070 090
- JP-A- 63 229 029
- US-A- 5 738 826
- US-A- 6 108 570
- AWAYDA MOUHAMED S ET AL: "Frequency-dependent capacitance of the apical membrane of frog skin: Dielectric relaxation processes" BIOPHYSICAL JOURNAL, vol. 76, no. 1 PART A, January 1999 (1999-01), pages 219-232, XP002336412 ISSN: 0006-3495
- HILLYARD STANLEY D ET AL: "K+ transport and capacitance of the basolateral membrane of the larval frog skin" AMERICAN JOURNAL OF PHYSIOLOGY, vol. 273, no. 6 PART 1, December 1997 (1997-12), pages C1995-C2001, XP002336413 ISSN: 0002-9513

## Description

### Technical Field

The present invention relates to a method and an apparatus for measuring the human skin surface condition. More particularly, the present invention relates to a method of measuring the electrical potential condition of the human skin surface simply from the electrical potential difference between the skin surface and a sublingual, without giving pain to human, and an apparatus which is suitable for practicing the method.

### Background Art

The skin surface of a human receives various kinds of stimulation from the natural world. When the skin surface receives stimulation, a symptom appears on the skin, and at the same time, an itch, a pain, a smarting pain and the like which are expressed by human senses are affected in many cases. Also in many cases, no symptom appears but only the sense is produced. Such stimulation is pleasant in some cases, but are unpleasant in many cases. For example, stimulation such as an itch is unpleasant to human. Such stimulation belongs to the category of human senses and is a nuisance because other people do not feel or see it.

On the other hand, since there has been no method for measuring the skin surface condition, it was necessary to rely on human appeal to measure the degree of senses. Accordingly, if such stimulation could be substituted by numeric values and the like, which are objective, it would be possible to share senses, which are subjective, and furthermore, if the above numerical values and the like are used, rapid development of drugs for inhibiting unpleasant stimulation can be expected.

In addition, as described above, information about the human skin surface condition is useful in the field of dermatology. In the past, upon measuring the potential condition of the human skin surface, the dermis layer and the skin which has been vigorously rubbed with a file have been used as a standard. The latter case was defective in that the potential of a standard point changes in time, and in both cases, a severe disorder was imparted to the skin due to opening in the skin a hole reaching to the dermis layer, or rubbing the skin.

WO 00/01301 discloses a method and an apparatus for measuring the human skin surface condition by means of evaluating the skin resistance or impedance variations in order to estimate the state of health of the internal organs of a human. The resistance or impedance is measured by allowing an electricity current to flow through the human body between the measurement electrode, which is placed in contact with the relevant skin area corresponding to the internal organ, and the reference electrode, which is placed in contact with any other skin area, usually the hand of the subject.

In view of the aforementioned circumstance, an objective of the present invention is to provide a method and an apparatus for simply measuring the electrical potential condition of the human skin surface without giving pain to the human.

### Disclosure of the Invention

In order to solve the aforementioned problem, the present inventors studied intensively, and as a result, paid attention to the human skin surface potential and discovered that the aforementioned problems can be solved by making a simple skin model by measuring the electrical potential condition of the skin surface using the sublingual potential as a standard, and led to the completion of the present invention.

More specifically, the present invention provides a method measuring the human skin surface condition, characterized by using a sublingual potential as a standard potential upon measuring of the electrical potential condition of the skin surface.

In order to practice this measuring method, there can be adopted a method for measuring the human skin surface condition from a difference in the electrical potentials measured by a standard electrode part communicating with a sublingual part and a measuring electrode part communicating with the skin surface.

Furthermore, the present invention is the skin surface condition measuring apparatus, which is provided with a standard electrode part adapted to facilitate communication to a sublingual part, a measuring electrode part communicating with the skin surface, and a potential difference detecting part for detecting an electrical potential difference between the standard electrode part and the measuring electrode part.

It is preferable that a standard electrode part used in this skin surface condition measuring apparatus using a sublingual potential as a standard potential is provided with an inner tube accommodating an internal solution with a standard electrode immersed therein, and an outer tube accommodating an internal solution which accommodates this inner tube to form a double tube, and a liquid junction is attached to the wall of the inner tube.

In this case, communication between the sublingual part and the standard electrode part is made by a salt bridge connecting the sublingual part and the internal solution in the outer tube of the standard electrode part.

In addition, the measuring electrode part used in the skin surface condition measuring apparatus using a sublingual potential as a standard potential may be provided with an inner tube accommodating an internal solution with a measuring electrode part immersed therein, and an outer tube accommodating an internal solution which accommodates this inner tube to form a double tube, and a liquid junction attached to the wall of the inner tube.

In this case, communication between the skin surface and the measuring electrode part is made by a salt bridge connecting the skin surface and the internal solution in the outer tube of the measuring electrode part.

The skin surface condition using said sublingual potential of the present invention as a standard potential may be an electrical potential condition of the skin surface.

According to the skin surface condition measuring method and apparatus using a sublingual potential as a standard potential, by using a suitable apparatus to measure the electrical potential difference between the skin surface and the sublingual part, the skin surface condition can be measured simply without causing pain to a human.

Note that, in the present invention, the concept of the skin surface condition includes, in addition to the potential condition of skin surface, various changes in condition occurring on skin surface, for example, the change in condition and the degree thereof due to stimulation, inhibition of stimulation and the like.

### Brief Description of the Drawings

Fig. 1 is a diagram showing an embodiment of the human skin surface condition measuring apparatus using a sublingual potential as a standard potential according to the present invention.

Fig. 2 is a graph showing an example employing the human skin surface condition measuring apparatus using a sublingual potential as a standard potential according to the present invention.

Fig. 3 is a graph showing the correlation between a human skin surface potential employing the human skin surface condition measuring apparatus using a sublingual potential as a standard potential according to the present invention, and a human skin surface potential according to the conventional method.

### Best Mode for Carrying Out the Invention

Embodiments of the present invention will be described in detail below.

A method and apparatus for measuring the human skin surface condition using a sublingual potential as a standard potential will be explained.

In this method of the present invention, a sublingual area is used as a standard to measure the difference in the electrical potentials (mV) of the skin surface and the sublingual area. As a result, the skin surface condition is measured.

Next, an apparatus which is suitably used in the aforementioned method using a sublingual potential as a standard potential of the present invention will be explained with reference to the drawings.

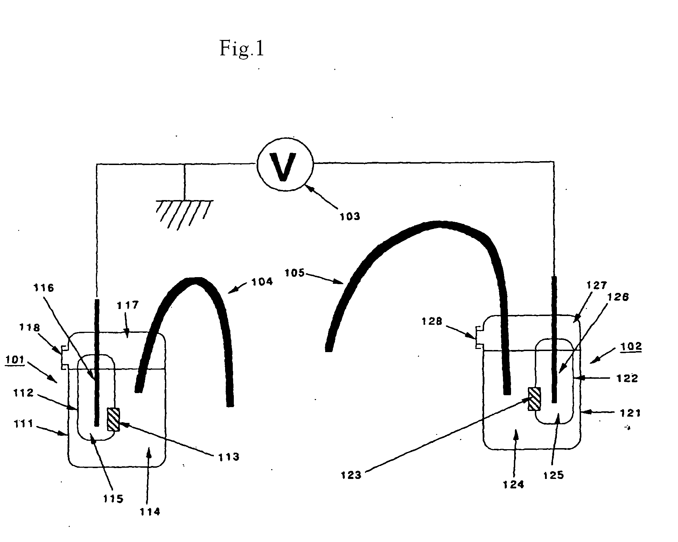

Fig. 1 shows an example of the human skin surface condition electrical potential condition of the skin surface measuring apparatus using a sublingual potential as a standard potential in the present invention. As shown in Fig. 1, the human skin surface condition measuring apparatus has a standard electrode part 101 communicating to a sublingual area (not shown), a measuring electrode part 102 communicating to the skin surface (not shown), and a potential difference detecting part 103 for detecting a potential difference between the standard electrode part 101 and the measuring electrode part 102.

The standard electrode part 101 has double outer tube 111 and inner tube 112 made of a plastic, and a tube wall of the inner tube 112 has a liquid junction 113 comprising a porous ceramic having the function of not permeating a solution but permeating electrons or ions. The interiors of the double outer tube 111 and inner tube 112 are filled with internal solutions 114, 115 comprising a KC1 solution having the prescribed concentration, respectively, and a standard electrode 116 composed of silver-silver chloride is immersed in the internal solution 115 of the inner tube 112. This standard electrode 116 is attached to an electrode holder 117 which also functions as a cap and closes upper ends of the double outer tube 111 and inner tube 112, and in the condition where the electrode is suspended from there via a conductor, the electrode is immersed in the internal solution 115 in the inner tube 112. A solution supplementing port 118 for supplementing the internal solution 114 in the outer tube 111 is provided on a circumferential plane near an upper end of the outer tube 111.

In addition, in the standard electrode part 101 in Fig. 1, the double outer tube 111 and inner tube 112 all made of glass may be used. In addition, as the liquid junction 113, the known liquid junctions such as solutions having the pseudo-crystal structure such as gelatin, agar and the like and liquid junctions including those used as a salt bridge such as porous Bicoll glass and the like may be used. Alternatively, as the standard electrode 116, an electrode comprising calomel may be used. In addition, as the internal solution 115, a solution of chloride such as sodium chloride, lithium chloride and the like can be used in addition to a KCl solution. As the internal solution 114 in the outer tube 111, physiological saline may be used in addition to chloride used in the internal solution 115 in the inner tube 112.

The measuring electrode part 102 has the similar structure to that of the standard electrode part 101. That is, the measuring electrode part 102 has double outer tube 121 and inner tube 122 made of a plastic, and a tube wall of the inner tube 122 has a liquid junction 123 composed of a porous ceramic. The interiors of the double outer tube 121 and inner tube 122 are filled with internal solutions 124, 125 comprising a KCl solution having the prescribed concentration, respectively, and a measuring electrode 126 composed of silver-silver chloride is immersed in the internal solution 125 of the inner tube 122. This measuring electrode 126 is attached to an electrode holder 127 which also functions as a cap and closes upper ends of the double outer tube 121 and inner tube 122, and in the condition where the electrode is suspended from there via a conductor, the electrode is immersed in the internal solution 125 in the inner tube 122. A solution supplementing port 128 for supplementing the internal solution 124 in the outer tube 121 is provided on a circumferential plane near an upper end of the outer tube 121.

Note that the measuring electrode part 102 in Fig. 1 can also take the similar embodiment to that of the standard electrode part 101. That is, the double outer tube 121 and inner tube 122 all made of glass may be used. In addition, porous Bicoll glass may be used in the liquid junction 123. In addition, as the measuring electrode 126, an electrode comprising calomel may be used. In addition, as the internal solution 125, a solution of chloride such as sodium chloride, lithium chloride and the like can be used in addition to a KCl solution. As the internal solution 124 in the outer tube 121, physiological saline may be used in addition to chloride used in the internal solution 125.

Furthermore, the standard electrode part 101 in Fig. 1 is communicated to a sublingual area via a salt bridge 104 immersed in the internal solution 114 in its outer tube 111. The salt bridge 104 may be the known one, and for example, flexible plastic tubes, rubber tubes such as silicone and the like, and the like which are filled with agarose containing physiological saline are used.

In addition, the measuring electrode part 102 is communicated to the skin surface via a salt bridge 105 immersed in the inner solution 124 in its outer tube 121. The salt bridge 105 may be the known one, and for example, flexible plastic tubes, rubber tubes such as silicone and the like, and the like which are filled with agarose containing physiological saline are used.

The standard electrode 116 of the standard electrode 101 and the measuring electrode 126 of the measuring electrode part 102 are connected to the potential difference detecting part 103, respectively.

In order to measure the human skin surface condition (electrical potential condition) using the aforementioned apparatus, one end of a salt bridge, the other end of which is communicated to a standard electrode part, is placed in mouth of a subject to be measured, and is fixed sublingual. Upon this, one end of a salt bridge is not directly placed in mouth, but a tube such as a plastic, a silicon rubber and the like containing therein a cotton or the like containing exchangeable physiological saline is connected to an end of a salt bridge, and the tube may be placed in mouth and fixed sublingual. By doing so, since the tube can be exchanged every subject to be measured, there is no unclean feeling, and in particular, the tube is effective upon use at shop fronts and clinical places. On the other hand, after one end of a salt bridge, the other end of which is communicated to a measuring electrode part, is contacted with and fixed on the skin surface of a subject to be measured, a potential difference between the standard electrode part and the measuring electrode part is measured with a potential difference detecting part. Note that it is preferable that communication between the salt bridge and the skin surface is through a filter in order to contact both uniformly.

According to the method for measuring the skin surface condition using a sublingual potential as a standard potential, a number of information which are useful for dermatology can be obtained. For example, physical stimulation such as barrier destruction with a cellophane adhesive tape and the like is given to the skin surface, or chemical stimulation is given to the skin surface by coating a stimulating substance such as neuropeptide, histamine, surfactant, organic acid, capsaicin and the like, an electrical potential of the skin surface having subjective sense such as itching and the like is measured, and the skin surface condition can be measured by expressing a change in the skin surface condition as an objective numerical value from comparison of electrical potential differences before and after stimulation.

Furthermore, an electrical potential difference is measured by coating a drug on the stimulated skin surface, and the degree of inhibition of skin stimulation by a drug may be measured from a value of a potential difference changed by drug coating.

Then, more specific Examples of the present invention will be explained.

### (Example 1)

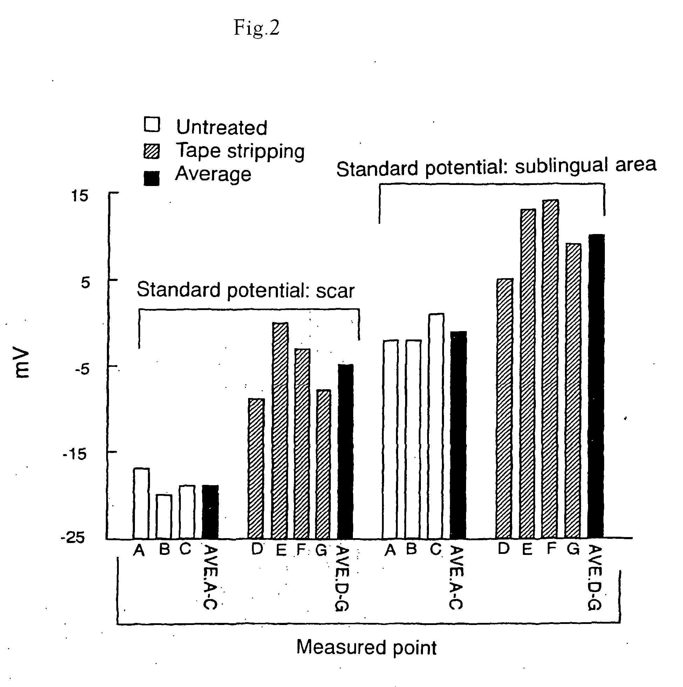

The electrical potential differences of skin surface relative to a sublingual area and a scar on the skin (epidermis) were measured using the skin surface measuring apparatus shown in Fig. 1. The skin used in measurement is a skin in a state of 2 hours after skin surface was barrier-destructed (tape stripped) with a cellophane adhesive tape. Fig. 2 shows electrical potentials at several places of skin using each of a sublingual potential and a skin scar potential as a standard potential.

As shown in Fig. 2, a potential rises 2 hours after tape stripping. It can be seen that this rise in potential is detectable using either a sublingual area or a skin scar as a standard. In addition, letters A to G in the figure denote measurement points, and AVE. denotes an average value.

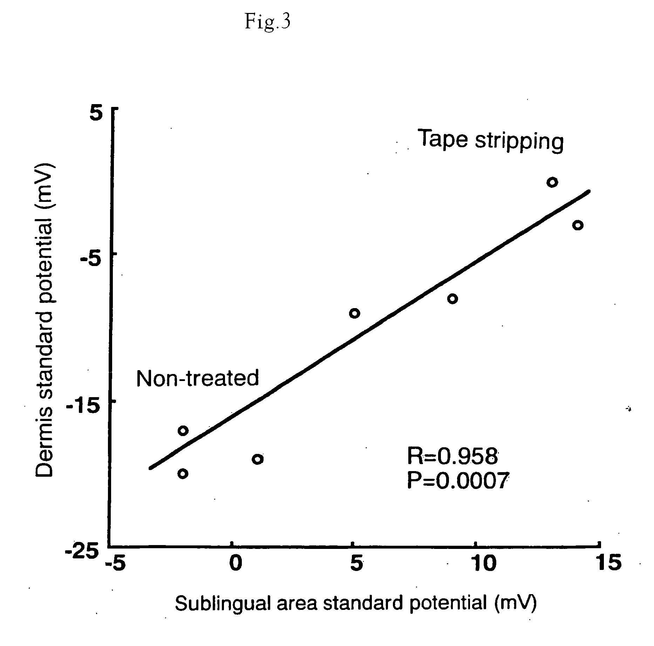

Furthermore, Fig. 3 plots the results of Fig. 2 in connection with the relationship between a skin surface potential using a skin scar as a standard and a skin surface potential using a sublingual area as a standard. It can be seen that there is significant correlation between both potentials, and also when a sublingual area is used as a standard in place of the previous method using a skin scar as a standard, a skin surface potential can be stably measured.

### Industrial Applicability

As explained above, according to the human skin surface condition electrical potential condition) measuring apparatus, which uses a sublingual potential as a standard, the human skin surface condition can be measured simply without imparting pain to a human. From this result, a number of useful applications can be expected such as confirmation of the degree of stimulation to be imparted to skin, investigation of an inhibiting substance to skin stimulation from a change in a potential difference by coating a substance for warding off stimulation on skin, and the like.

## Claims

1. A method for measuring the human skin surface condition, which comprises using a sublingual potential as a standard potential upon measuring the human skin surface condition.

2. The method for measuring the human skin surface condition according to claim 1. which comprises measuring the human skin surface condition from a difference in the electrical potentials measured by a standard electrode part communicating with a sublingual area and a measuring electrode part communicating to the skin surface.

3. The method for measuring the human skin surface condition according to claim 1 or 2. wherein the skin surface condition is the electrical potential condition of the skin surface.

4. A human skin surface condition measuring apparatus adapted to carry out the method of claims 1 to 3, which apparatus comprises (a) a standard electrode part adapted to facilitate the communication to the sublingual area, (b) a measuring electrode part communicating to the skin surface, and (c) an electrical potential difference detecting part for detecting a difference in the electrical potentials measured by said standard electrode ' part and said measuring electrode part.

5. The human skin surface condition measuring apparatus according to claim 4, wherein the standard electrode part is provided with an inner tube accommodating an internal solution with a standard electrode immersed therein, and an outer tube accommodating an internal solution which accommodates the inner tube to form a double tube, and a liquid junction is attached to the wall of said inner tube.

6. The human skin surface condition measuring apparatus according to claim 5, wherein communication between said sublingual area and said standard electrode part is made by a salt bridge connecting the sublingual area and the internal solution in the outer tube of the standard electrode part.

7. The human skin surface condition measuring apparatus according to any one of claims 4 through 6, wherein the measuring electrode part is provided with an inner tube accommodating an internal solution with a measuring electrode immersed therein, and an outer tube accommodating an internal solution which accommodates the inner tube to form a double tube, and a liquid junction is attached to the wall of said inner tube.

8. The human skin surface condition measuring apparatus according to claim 7, wherein communication between said skin surface and said measuring electrode part is by a salt bridge connecting the skin surface and the internal solution in the outer tube of the measuring electrode part.

9. The human skin surface condition measuring apparatus according to any one of claims 4 through 8, wherein the human skin surface condition is the electrical potential condition of the skin surface.

## Patentansprüche

1. Verfahren zur Messung des Oberflächenzustandes von menschlicher Haut, welches die Verwendung eines sublingualen Potentials als Standardpotential zur Messung des Oberflächenzustandes von menschlicher Haut umfasst.

2. Verfahren zur Messung des Oberflächenzustandes von menschlicher Haut nach Anspruch 1, welches das Messen des Oberflächenzustandes von menschlicher Haut aus einem Unterschied in dem elektrischen Potential, gemessen durch einen Standardelektrodenteil, der mit einem sublingualen Bereich in Verbindung steht, und einen Messelektrodenteil, der mit der Hautoberfläche in Verbindung steht, umfasst.

3. Verfahren zur Messung des Oberflächenzustandes von menschlicher Haut nach Anspruch 1 oder Anspruch 2, worin der Hautoberflächenzustand der Zustand des elektrischen Potentials der Hautoberfläche ist.

4. Vorrichtung zur Messung des Oberflächenzustandes von menschlicher Haut, welche zur Durchführung des Verfahrens nach einem der Ansprüche 1 bis 3 angepasst ist, wobei die Vorrichtung umfasst (a) einen Standardelektrodenteil, der zur Erleichterung der Verbindung mit dem sublingualen Bereich angepasst ist, (b) einen Messelektrodenteil, der mit der Hautoberfläche in Verbindung steht, und (c) einen den elektrischen Potentialunterschied detektierenden Teil zur Detektierung des Unterschieds in den elektrischen Potentialen, die der Standardelektrodenteil und der Messelektrodenteil messen.

5. Vorrichtung zur Messung des Oberflächenzustandes von menschlicher Haut nach Anspruch 4, worin der Standardelektrodenteil mit einem Innenrohr, welches eine interne Lösung mit einer darin eingetauchten Standardelektrode aufweist, und einem Außenrohr, welches eine interne Lösung aufweist, die das Innenrohr beinhaltet, unter Bildung eines Doppelrohrs, versehen ist und wobei ein Flüssigkeitsanschluss an der Wand des Innenrohrs vorgesehen ist.

6. Vorrichtung zur Messung des Oberflächenzustandes von menschlicher Haut nach Anspruch 5, worin die Verbindung zwischen dem sublingualen Bereich und dem Standardelektrodenteil mittels einer Salzbrücke vorgesehen ist, die den sublingualen Bereich und die interne Lösung in dem Außenrohr des Standardelektrodenteils verbindet.

7. Vorrichtung zur Messung des Oberflächenzustandes von menschlicher Haut nach einem der Ansprüche 4 bis 6, worin der Messelektrodenteil mit einem Innenrohr, das eine interne Lösung mit einer darin eingetauchten Messelektrode aufweist, sowie einem Außenrohr, welches eine interne Lösung aufweist, die das Innenrohr unter Bildung eines Doppelrohrs aufweist, versehen ist und wobei ein Flüssigkeitsanschluss an der Wand des Innenrohrs vorgesehen ist.

8. Vorrichtung zur Messung des Oberflächenzustandes von menschlicher Haut nach Anspruch 7, worin die Verbindung zwischen der Hautoberfläche und dem Messelektrodenteil mittels einer Salzbrücke vorgesehen ist, die die Hautoberfläche und die interne Lösung in dem Außenrohr des Messelektrodenteils verbindet.

9. Vorrichtung zur Messung des Oberflächenzustandes von menschlicher Haut nach einem der Ansprüche 4 bis 8, worin der Oberflächenzustand der menschlichen Haut der Zustand des elektrischen Potentials der Hautoberfläche ist.

## Revendications

1. Un procédé pour mesurer la condition de surface de peau humaine, comprenant une utilisation d'un potentiel sublingual en tant que potentiel standard en mesurant la condition de surface de peau humaine.

2. Le procédé pour mesurer la condition de surface de peau humaine selon la revendication 1, comprenant une mesure de la condition de surface de peau humaine à partir d'une différence dans le potentiel électrique mesuré par une partie d'électrode standard communiquant avec une aire sublinguale et une partie d'électrode de mesure communiquant avec la surface de peau.

3. Le procédé pour mesurer la condition de surface de peau humaine selon la revendication 1 ou 2, dans lequel la condition de surface de peau est la condition de potentiel électrique de la surface de peau.

4. Un appareil de mesure de la condition de surface de peau humaine adapté pour mettre en oeuvre le procédé des revendications 1 à 3, ledit appareil comprenant (a) une partie d'électrode standard adaptée pour faciliter la communication avec l'aire sublinguale, (b) une partie d'électrode de mesure communiquant avec la surface de peau, et (c) une partie de détection de différence de potentiel électrique pour détecter une différence dans les potentiels électriques mesurés par ladite partie d'électrode standard et ladite partie d'électrode de mesure.

5. L'appareil de mesure de la condition de surface de peau humaine selon la revendication 4, dans lequel la partie d'électrode standard est fournie avec un tube intérieur accueillant une solution interne avec une électrode standard immergée dans celle-ci, et un tube extérieur accueillant une solution interne qui accueille le tube intérieur pour former un tube double, et une jonction liquide est attachée au mur dudit tube intérieur.

6. L'appareil de mesure de la condition de surface de peau humaine selon la revendication 5, dans lequel la communication entre l'aire sublinguale et la partie d'électrode standard est faite par un pont salin reliant l'aire sublinguale et la solution interne dans le tube extérieur de la partie d'électrode standard.

7. L'appareil de mesure de la condition de surface de peau humaine selon l'une quelconque des revendications 4 à 6, dans lequel la partie d'électrode de mesure est fournie avec un tube intérieur accueillant une solution interne avec une électrode de mesure immergée dans celle-ci, et un tube extérieur accueillant une solution interne qui accueille le tube intérieur pour former un tube double, et une jonction liquide est attachée au mur dudit tube intérieur.

8. L'appareil de mesure de la condition de surface de peau humaine selon la revendication 7, dans lequel la communication entre la surface de peau et la partie d'électrode de mesure est faite par un pont salin reliant la surface de peau et la solution interne dans le tube extérieur de la partie d'électrode de mesure.

9. L'appareil de mesure de la condition de surface de peau humaine selon l'une quelconque des revendications 4 à 8, dans lequel la condition de surface de peau humaine est la condition de potentiel électrique de la surface de peau.
